# EUROPEAN PATENT SPECIFICATION

(11) **EP 4 171 469 B1**
(45) Date of publication and mention of the grant of the patent: **25.10.2023**
(21) Application number: 21745839.7
(22) Date of filing: 25.06.2021
(51) Int. Cl.: A61H 23/02, A61B 5/00, G06F 3/00, G16H 10/00, G16H 20/00

(54) **SYSTEMS FOR ASSOCIATING SYMPTOMS WITH MEDICAL CONDITIONS**
SYSTEME ZUR ASSOZIATION VON SYMPTOMEN MIT MEDIZINISCHEN ZUSTÄNDEN
SYSTÈMES D'ASSOCIATION DE SYMPTÔMES À DES ÉTATS MÉDICAUX

(30) Priority: 26.06.2020 WO PCT/IB2020/056091
(43) Date of publication of application: 03.05.2023
(73) Proprietor: Charco Neurotech Ltd, London E9 5EN (GB)
(72) Inventor: JUNG, Soo Min, Cambridge CB2 9DP (GB); PIERRES, Floyd, Cambridge CB2 9DP (GB)
(74) Representative: Basck Limited
(86) International application number: PCT/IB2021/055666
(87) International publication number: WO 2021/260640

(56) References cited:
- WO-A1-2017/210729
- US-A1- 2010 169 409
- US-A1- 2015 073 310
- US-A1- 2018 206 775
- US-A1- 2019 110 754
- US-A1- 2019 200 915
- US-A1- 2019 206 566

## Description

### TECHNICAL FIELD

The present disclosure relates generally to monitoring systems; more specifically, the present disclosure relates to systems for associating symptoms with medical conditions. Moreover, the present disclosure relates to methods for associating symptoms with medical conditions. Furthermore, the present disclosure relates to computer program products for executing the aforementioned methods.

### BACKGROUND

With the advancement in medical technologies, monitoring and managing progression of a disease has become easier. Typically, various medical devices (such as wearable devices or hand-held devices) are at the disposal of patients and medical professionals for tracking symptom progression and communicating with each other. Despite the availability of such monitoring devices, people fail to identify symptoms and associate the symptoms to a specific medical condition. The failure to associate one or more symptoms to a specific medical condition could be attributed to myriad common symptoms shared between a plurality of medical conditions. Specifically, it would be difficult to identify symptoms and associate symptoms with a specific medical condition for people suffering from neurological disorders (such as Parkinson's disease) or other conditions, such as old age, withdrawal symptoms, and the like. More specifically, the symptoms appear generic in nature and can be associated with a similar medical condition rather than the correct medical condition. For example, tremors may be associated with Parkinson's disease, essential tremors, or epilepsy.

Conventionally, clinically validated evaluation tools may be used to identify and associate symptoms with a medical condition. In this regard, healthcare professionals require the subject, i.e. a patient or any person experiencing symptoms, to provide inputs for evaluation thereof. However, such evaluation tools are labour intensive and may not be a comforting diagnosis method for most of the subjects. Moreover, even if the conventional evaluation tools enable efficient identification of symptoms and associated medical condition of the subject, such subjects lack access to tools, that are tailored to their requirements, for the management of their disease. Patients suffering from Parkinson's disease, particularly, face difficulties in tracking symptom progression and communicating with healthcare professionals due to infrequent and brief check-ups. Moreover, they often struggle to take medication on time and keep notes on their medication intake. Furthermore, due to the debilitating nature of the disease, small mistakes can lead to periods of heavily affected quality of life, and at times results in highly negative quality of life. Therefore, it is necessary to ensure that current treatment strategies can be supported by any innovation while providing a tool to allow patients to retain higher independence and quality of life.

Therefore, in light of the foregoing discussion, there exists a need to overcome the aforementioned drawbacks associated with conventional techniques of identifying and associating symptoms with a medical condition, improving the way that patients can manage their symptoms and disease, and communicating with their healthcare professionals for better quality of life.

US 2018/206775 A1 represents the background art.

### SUMMARY

The present invention provides a system according to claim 1. Further aspects and preferred embodiments are defined in the dependent claims. Aspects, embodiments, examples, and methods of the present disclosure that do not fall within the scope of the appended claims do not form part of the invention and are merely provided for illustrative purposes.

The present disclosure seeks to provide a system for associating a symptom with a medical condition. The present disclosure also seeks to provide an exemplary method for associating a symptom with a medical condition. The present disclosure further seeks to provide an exemplary computer program product comprising computer-readable instructions to be executed by a processing hardware of a computerized device. The present disclosure seeks to provide a solution to the existing problem of identifying correct symptoms and associating the identified symptoms with a correct medical condition. An aim of the present disclosure is to provide a solution that overcomes at least partially the problems encountered in prior art, and to provide an efficient and robust solution for associating the symptom experienced by a user with a medical condition.

In one aspect, the present disclosure provides a system for associating a symptom with a medical condition according to claim 1.

Embodiments of the present disclosure substantially eliminate or at least partially address the aforementioned problems in the prior art, and enable identifying and associating symptoms with a medical condition, and monitoring progression of symptoms by engaging the user in various activities, and increasing clinician interaction frequency of the user by sharing the user data with the clinicians routinely.

Additional aspects, advantages, features and objects of the present disclosure would be made apparent from the drawings and the detailed description of the illustrative embodiments construed in conjunction with the appended claims that follow.

It will be appreciated that features of the present disclosure are susceptible to being combined in various combinations without departing from the scope of the present disclosure as defined by the appended claims.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the present disclosure is not limited to specific methods and instrumentalities disclosed herein. Moreover, those skilled in the art will understand that the drawings are not to scale. Wherever possible, like elements have been indicated by identical numbers.

Embodiments of the present disclosure will now be described, by way of example only, with reference to the following diagrams wherein:
FIG. 1 is a flowchart of steps of method for associating a symptom with a medical condition, in accordance with an embodiment of the present disclosure;
FIG. 2 is an exemplary illustration of a system for associating a symptom with a medical condition, in accordance with an embodiment of the present disclosure
FIGs. 3, 4A, 4B, 4C, 5A, 5B, 5C, 6A and 6B are exemplary user interfaces on the user device, in accordance with an embodiment of the invention;
FIG. 7A is a cross-sectional view of an exemplary wearable device, in accordance with an embodiment of the invention; and
FIG. 7B is an exploded view of an exemplary wearable device, in accordance with an embodiment of the invention.

In the accompanying drawings, an underlined number is employed to represent an item over which the underlined number is positioned or an item to which the underlined number is adjacent. A non-underlined number relates to an item identified by a line linking the non-underlined number to the item. When a number is non-underlined and accompanied by an associated arrow, the non-underlined number is used to identify a general item at which the arrow is pointing.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practising the present disclosure are also possible.

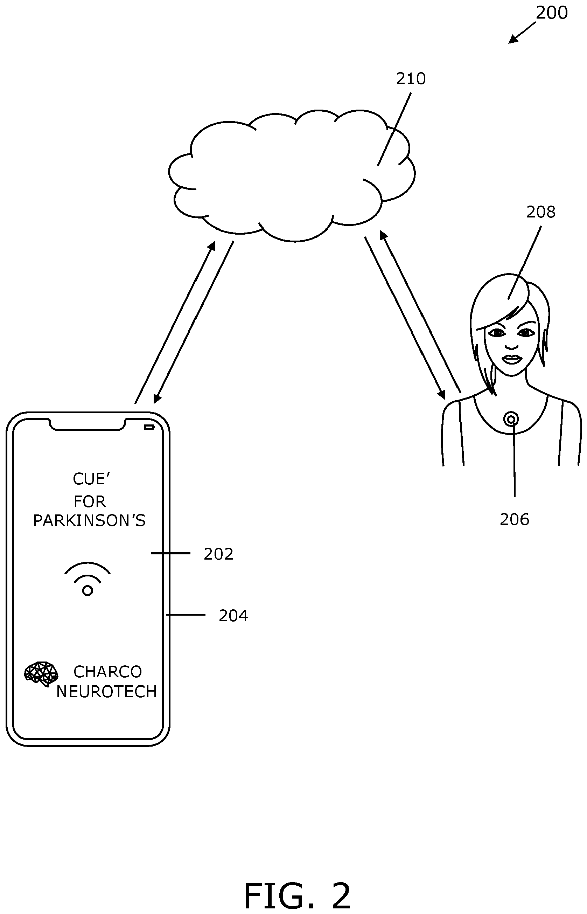

In some embodiments, a wearable device is configured for tracking by an application software, when in operation, as shown in Figure 2 The wearable device has a to-use application software that is integrated with an external device (referred to as "*user device*" hereafter). Such to-use application software integrated in at least one compatible user device is referred to as a device integration application. A compatible user device is configured to run the device integration application thereon. The user device includes, but is not limited to, a mobile, a laptop computer, a tablet computer, a desktop computer, a palmtop computer and a smart watch. The term "*device integration application*" refers to a software program for execution by the user device by employing a processor of said user device. Notably, the device integration application comprises a set of predefined functions that are programmed to provide instructions to hardware and/or software elements of the user device. Furthermore, the device integration application is configured to provide a user interface on a display of the user device, to allow the user to perform specific associated tasks. In an example, the device integration application is an application programming interface. In an embodiment, the device integration application is affiliated to an organisation. Therefore, the device integration application functions in accordance with pre-programmed guidelines provided by the organisation. The device integration application is configured to function in accordance with the pre-programmed guidelines upon installation thereof.

In some embodiments, the device integration application enables the user to apply touch or light pressure as a cue to access the user input when the wearable device is being worn or is adhered to the user (i.e. when in operation). The touch or light pressure application by the user on the user input is configured to provide instructions to a controller of the wearable device to instruct at least one stimulating element to provide an output based on the received user input. In some embodiment, the device integration application enables the user to instruct the user device when to remind them to take prescribed medicines. In this case, the device integration application includes prescription information related to, for example, names of medicines, doses of medicines, time of taking medicine, and so on. Moreover, the device integration application allows configuring the wearable device based on user's requirement (in other words, is tailored to meet specific requirements). In such embodiment, the device integration application enables producing an identifiable alert in addition to a stimulation alert. Beneficially, the identifiable alert comprises an alert pattern, such as visual, audible, or a combination thereof, for family or carer of the user to help an incapacitated user. In some embodiments, the device integration application allows scheduling appointments with a doctor and sending an advanced notification (or reminder) therefor. It will be appreciated that an advanced notifications may be generated based on a predefined period, for example, 2 days, 1 day, 6 hours, and 2 hours before a due appointment.

In some embodiments, the device integration application enables tracking and recording regular use of the wearable device, symptoms, variations and progression of the symptoms, and the efficacy of the wearable device as well as therapies (including medicines, meditation, etc.) on the symptoms. In some embodiments, the device integration application is configured to collect and analyse data recorded by the user and use it to measure and provide improvement as graphs. In some embodiments, the device integration application reports (for example sends via email or another device integration application) day-to-day analysis of the wearable device to the user's doctor or physician via a communication network. Additionally, or alternatively, the device integration application triggers an automatic alert when readings go beyond a predefined threshold. In an alternate embodiment, the wearable device is operable to be used without any Bluetooth^{®} connection. The wearable device comprises a first command, i.e. touch or pressure application once for 5 minutes stimulation, and a second command for continuous stimulation. In such embodiment, the wearable device, through the device integration application, is configured to provide a medication alert, as well as manage the alert system, vibration amplitude, duty cycle, vibration wave form, battery status and on/off of the wearable device. In some embodiments, the device integration application is used to trigger the wearable device from a remote location, for example by the user's doctor, carer, or a family member authorised to access the wearable device in an emergency situation. It will be appreciated that the wearable device is a stand-alone device and requires no new software or hardware for its functioning.

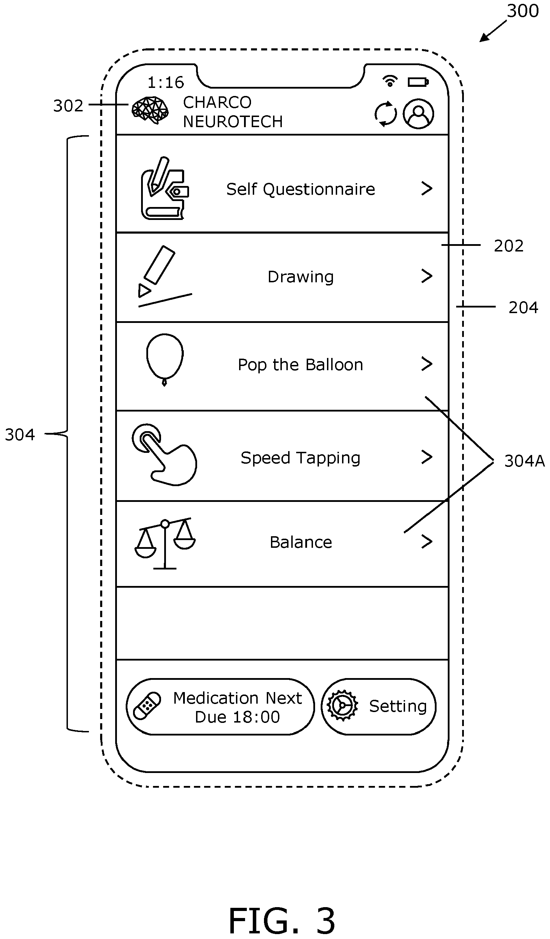

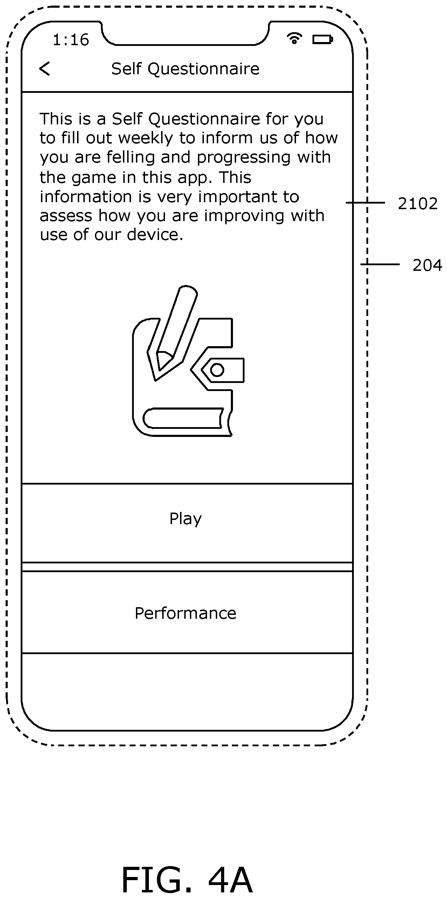

Figure 3 is an exemplary implementation of a successful installation of the device integration application on a user device, such as a smart phone. As shown, a logo or icon of the device integration application is visible on the smart phone display. The device integration application comprises a main menu with a plurality of options (as shown in detail in Figures 4A, 4B, 4C, 5A, 5B, 5C, 6A and 6B), such as a variety of games, self-assessment, the medication alert, and so on. The device integration application allows toggling between the various options on the main menu by going back from one option to the main menu and selecting another option. The variety of games are designed to test speed and accuracy of the user, such as a patient suffering from Parkinson's disease. In an example, one of the variety of games include drawing a spiral and/or a straight line between the two points shown on the screen using a finger. The user can take multiple attempts that helps in improving movement. In another example, one of the variety of games include identifying and tracking a change in the display, for example, popping as many balloons as possible within a time limit using the finger, tapping alternating dots on the screen as fast as possible within a time limit using the finger, and so forth. The self-assessment comprises a questionnaire for the user. The user is required to fill out the self-assessment questionnaire in routine, for example weekly, to track and inform doctor or carer about the user's health. The questionnaire may require the user to choose from a predefined scale of responses ranging for example from "None, Slight, Moderate, Severe and Unable" to answer for questions such as "problems with walking", "problems with washing or dressing"," problems with daily activities", "pain/discomfort", "anxiety/depression", and so forth. The device integration application allows the user to move to a new question after registering an answer for the present question. Moreover, the device integration application allows the user to evaluate an overall health (quality of life (QOL)) on a scale of 0 to 100, where 0 is the worst and 100 is the best health the user can imagine. Furthermore, the device integration application provides the Medication Alerts, such as the medication next due, and a time in 12- or 24-hour format, for the medication. Additionally, a log is provided for the user to register an answer (in a Yes or a No) if the said alert was addressed and the medication was taken due for that time. Furthermore, a Settings icon allows the user to set alarms, provide prescription details (i.e. medicines and doses) and the frequency for each medicine. Beneficially, the device integration application enables sending the data recorded by the user to respective physicians for tracking the progress of the user.

In one aspect, the present disclosure provides a system for associating a symptom with a medical condition, the system comprising a server arrangement, associated with a user device, the server arrangement is operable to:
(a) provide, to the user device, a set of user activities;
(b) receive, from the user device, information relating to a selection of a user activity from the set of user activities;
(c) receive, from the user device, a user input associated with the selected user activity;
(d) analyse the user input and assign a performance score thereto;
(e) identify, based on the performance score, one or more symptoms; and
(f) assign a severity score to the symptom for associating the symptom with a medical condition.

In another aspect, the present disclosure provides an exemplary method for associating a symptom with a medical condition using a system, the method not being part of the invention and comprising operating a server arrangement associated with a user device:
(a) providing, to the user device, a set of user activities;
(b) receiving, from the user device, information relating to selection of a user activity from the set of user activities;
(c) receiving, from the user device, a user input associated with the selected user activity;
(d) analysing the user input and assigning a performance score thereto;
(e) identifying, based on the performance score, one or more symptoms; and
(f) assigning a severity score to the symptom for associating the symptom with a medical condition.

In yet another aspect, the present disclosure provides an exemplary computer program product not being part of the invention and comprising a non-transitory computer-readable storage medium having computer-readable instructions stored thereon, the computer-readable instructions being executable by a computerized device (namely, one having server arrangement as mentioned above) comprising processing hardware to execute the aforementioned method.

The present disclosure provides the aforementioned system and method for associating a symptom with a medical condition. In this regard, the method uses the system to provide a set of user activities, such as a self-assessment test, a game and so on, to encourage a user, such as user (or patient) experiencing the symptom, to provide a user input, such as health-related data or play a game and so on, that is analysed to identify the symptoms and associate the identified symptom with the medical condition. The method is implemented using for example an application software or app, for encouraging a user to provide health-related data and/or engage physically in order to associate symptoms with medical conditions and routinely monitor the progression of symptoms associated with the medical condition. The method further enables recording the progression of symptoms and assessment thereof, such as by a healthcare professional, carer or researcher, to manage overall health (quality of life) of the user. Moreover, the method assists the user in following the prescribed measures even when a wearable device for alleviating the symptoms associated with the medical condition, is not in use or without the need of an additional specialised tool. The method enables symptom tracking and allows the user (or carers thereof) to adjust settings of the associated devices (i.e. the user device or the wearable device coupled to the user device) such as medication alerts, notifications, and so on, to help alleviate the current lack of long-term support available to the users. The present disclosure also provides the aforementioned system using the aforementioned method as a standalone solution requiring no complex arrangement, and configurable based on the user's requirement. Beneficially, the user-centric design provides the users with greatest benefits, such as improved social participation, and a better quality of life for people with Parkinson's disease for example.

Throughout the present disclosure, the term "*medical condition*" as used herein refers to an abnormal condition that negatively affects a part or whole of a structure or a function associated therewith in a person, namely a subject. Optionally, the medical condition is associated with at least one of: a neurological disorder, a muscular disorder, a neuro-muscular disorder, a cardiac disorder, a pulmonary disorder, a vision-related disorder, a speech-related disorder, and so forth. The medical condition may typically be caused by external factors, such as pathogens for example, or by internal dysfunctions, such as immunodeficiency for example. Optionally, the medical condition is Parkinson's disease, Alzheimer's disease, multiple sclerosis, neural infection, a shock, a stroke, neurodegenerative disease, or any other neurological disorder. The medical condition is typically associated with specific symptoms. It will be appreciated that a medical condition may be associated with a set of symptoms. Optionally, the two or more symptoms of the set of symptoms may be interrelated or diverse. In an example, a Parkinson's Patient may experience tremors, stiffness, slowness of movement, soft or slurred speech, and so on.

Optionally, the symptoms associated with the medical condition is at least one of: a bradykinesia, a dyskinesia, a tremor, an akinesia, a hypokinesia, a micrographia, a hypophonia, a tachyphemia, shaking, slowness, freeze of gait, pain, dysfunction, distress, social problems, depression, anxiety, and so on. The term "*bradykinesia*" as used herein refers to a slowness of movement and is one of cardinal manifestations of Parkinson's disease. The term "*dyskinesia*" as used herein refers to an involuntary, erratic, writhing movements of one or more body parts (such as face, arms, legs, trunk, and so forth) of a subject. Dyskinesia could cause rapid jerks or slow and extended muscle spasms. The term "*tremor*" as used herein refers to an involuntary, rhythmic, oscillatory movement of one or more body parts. Tremors may also be associated with age, overuse and withdrawal of drugs or alcohol, hypoglycemia, hyperthyroidism, increased stress, and so forth. The tremors are typically associated with muscle contraction and relaxation. The term "*akinesia*" as used herein refers to a loss or impairment of the power of voluntary movement. The term "*hypokinesia*" as used herein refers to a movement disorder where the movements have a 'decreased amplitude' as compared to a normal movement. Micrographia refers to a decreased size of handwriting. Hypophonia refers to reduced volume and tachyphemia refers to acceleration of speech segments. Moreover, the symptoms could be associated with the medical condition and monitored by analysing at least one of: a physiological state, a psychological state, a behavioural state, and lifestyle of the person experiencing the symptoms.

Throughout the present disclosure, the term "*server arrangement*" *as* used herein refers to a structure and/or module that includes programmable and/or non-programmable components configured to store, process and/or share information. Specifically, the server arrangement includes any arrangement of physical or virtual computational entities capable of enhancing information to perform various computational tasks. Furthermore, it should be appreciated that the server arrangement may be both single hardware server and/or plurality of hardware servers operating in a parallel or distributed architecture. In an example, the server arrangement may include components such as a memory, a processor, a network adapter and the like, to store, process and/or share information with the user device or other computing components.

Furthermore, the server arrangement is communicably coupled with the user device, associated with a user, such as a patient, or any other person using the user device. Specifically, the server arrangement may be communicably coupled with the user device using a wired and/or a wireless data communication network. In an example, the communication network includes but not limited to, a cellular network, short range radio (for example, such as Bluetooth^{®}), Internet, a wireless local area network, and an Infrared Local Area Network, or any combination thereof. Optionally, the server arrangement may be within the user device. Optionally, the user device is a mobile phone or a smart phone.

In an example, at an implementation level, the method of the present disclosure may be performed by the application software. The application software is a computer program or a group of programs designed to run (locally or through a web browser) on the user device associated with the user. Optionally, the application software may be affiliated to an organization, for example a hospital or health provider service. Therefore, the application software functions in accordance with pre-programmed guidelines provided by the organization. The application software is configured to function in accordance with the pre-programmed guidelines upon installation thereof. The application software is designed to suit the compatible device, such as android or iOS. Moreover, the application software is designed to help users to perform an activity, such as health management, entertainment or sport engagement, and so on, for which the application software can manipulate at least one of: a text, numbers, an audio, graphics or any combination thereof. In this regard, the user device provides a user interface on the display thereof for executing the application software. Optionally, the user interface is driven by a mobile or web application. Specifically, the mobile or web application logs in to display of the user device to access the user interface. Moreover, the mobile or web application enables sending commands to the server arrangement to configure the user device for achieving one or more functions of the user device.

The method comprises operating the server arrangement for providing, to the user device, a set of user activities. The set of user activities is provided as a list on the display of the user device. Typically, the set of user activities enable assessing one or more user parameters selected from at least one of: dexterity, focus, mobility, speech, lifestyle, a location, and so on. The set of user activities are entertaining and enable measuring the quality of life of the user. In this regard, the set of user activities assess the one or more user parameters in response thereto, identifying symptoms corresponding to the one or more parameters, associating symptoms with the medical condition, and determining progression of the symptom in the user. Optionally, the set of user activities is at least one of: a self-assessment questionnaire form, a motor gaming task, a voice-controlled gaming task, a charting task, and so forth. Moreover, the set of user activities could be used to gather information and generate data in the form of graphs, reports, day-to-day analysis, and so on, therefrom for analysis by a healthcare professional, a family or a researcher, to identify symptoms, medical condition and/or monitor progression of symptoms in the user.

Moreover, the method comprises operating the server arrangement for receiving, from the user device, data relating to selection of a user activity from the set of user activities. The user is required to select from the set of user activities provided on the display of the user device a user activity based on the user's interest or prescribed regimen. The user may toggle between the displayed set of user activities to select the user activity. The selected user activity is communicated to an app hosting server (and a server arrangement thereof) to load data associated with the selected user activity. It will be appreciated that the term "data relating to selection" relates to a choice of the user activity, wherein the selection could be made by means of a touch (such as by using a finger tap, a stylus pen or an ergonomic pen associated with the user device), a voice control, a gesture, a stare, and so forth. Optionally, selection is by means of finger tap on the display of the user device.

Furthermore, the method comprises operating the server arrangement for receiving, from the user device, a user input associated with the selected user activity. The user input is provided on the user interface using an input means, such as a touch (for example by using an index finger of the user, a stylus pen or an ergonomic pen associated with the user device), a microphone, an image sensor (associated with a camera of the user device), and so on. More optionally, the user input is provided as a text information, selecting a suitable option from an available list of options, an audio, a video, a graphics, a gesture, or a combination thereof. Optionally, the user input is provided based on the selected user activity. Optionally, the user input is at least one of: a health-related information of a user, an inertial information from the user, a medication data provided to the user, and so forth.

Optionally, the selected user activity is a motor gaming task, that requires the user input as a motor movement, and wherein the motor gaming task is selected from at least one of: a speed tapping game, a balancing game, a maze game, a puzzle game, an interactive cognitive game, a cognitive task. The term *"motor gaming task"* as used herein refers to one or more games that require one or more motor movements, i.e. touch-based user inputs, in response to the selected user activity. In an example, the motor movement is selected from: a finger tap, a finger swipe, an angular movement of the finger, a displacement of the finger, a drag using one or more fingers, or a combination thereof. It will be appreciated the motor movement could be a movement generated using a hand, an arm, a leg, a neck, eyes, or any other body part alone or in combination, based on the selected user activity. Optionally, the user input is a visuomotor feedback. Optionally, the motor gaming task is a typical engagement activity specifically designed to test speed and accuracy of the user. Moreover, the motor gaming task comprises multiple skill levels for user participation.

In an example, the motor gaming task is a cognitive task, such as drawing a spiral and/or a straight line between the two points shown on the screen. In this regard, the user input is use of a finger or a stylus pen or an ergonomic pen. It will be appreciated that spiral drawing is a clinically validated method for analysing a hand motor movement of a subject, such as a patient. Conventionally, the spiral drawing is done on a paper for diagnosing patients for diseases like Parkinson's disease, essential tremor, and others.

In another example, the motor gaming task is speed tapping, that requires identifying and tracking a change in the display of the user device within a time limit using the finger or stylus pen, for example, by popping balloons , tapping alternating dots, aligning one or more items along a straight line (horizontally or vertically), and the like. In yet another example, the motor gaming task is an interactive cognitive game, such as an infinite (endless) runner game, strategy games (such as action or adventure games), sports-based games (such as chess). In still another example, the motor gaming task is a balancing game that includes user device balancing test. Typically, the balancing games uses device accelerometers to determine one or more of a dexterity or focus of the user while holding the device. Optionally, the balancing game may be a physical balancing game where the user device is balanced on a palm, hand, leg, and other body parts of the user. Alternatively, the balancing game may be a virtual balancing game where the user balances weights on the display of the user device using a touch-based input. Beneficially, the present disclosure provides gamified versions of the clinically validated evaluation tools, for identifying various motor-related symptoms and associated medical condition therewith, to encourage user interactions. Moreover, other cognitive tasks may include a writing task, an element matching task, a free-hand drawing, tracking a guiding point with a defined velocity, and the like. Additionally, beneficially, gamifying various evaluation tools help entertain the users and fight psychological isolation.

Optionally, the selected user activity is a voice-controlled gaming task, that requires the user input as at least one of: a vocal input, a motor movement. The term *"voice-controlled gaming task"* as used herein refers to one or more games that require a voice-based user input, in response to the selected user activity. Optionally, the voice-controlled gaming task may require vocal input in combination with a visual feedback, a motor movement, and the like. In an example, the voice-controlled gaming task is controlling a guiding point using a voice modulation in the vocal input. It will be appreciated that the vocal input may be a humming sound, a whistle, a command, or a combination thereof. Optionally, the voice-controlled gaming task is a typical engagement activity specifically designed to test speech and vocal control of the user. Moreover, the voice-controlled gaming task comprises multiple skill levels for user participation.

It will be appreciated that different types of games may be provided to the user device for encouraging the user to participate and monitor the symptoms and progression thereof. Moreover, the different types of games are therapeutic games that may be gamified versions of clinically (or therapeutically) validated evaluation tools. The different types of games include, but do not limit to, the above-mentioned examples, and may be varied in terms of a background view, a background score, speed, skill levels, total time duration, types of avatars (namely, characters) of the game, types of hurdles, and so forth. Moreover, it will also be appreciated that different types of games are associated with different types of user input. For example, the spiral drawing test requires the user to follow an Archimedes spiral using the index finger, the stylus pen or the ergonomic pen. Moreover, the user can take multiple attempts at the selected at least one user activity to aid in improving accuracy. Optionally, the games may be played at multiple skill levels, such as three, four, five, seven, ten, and so on, and with different background images, to increase the user's participation and engagement. Furthermore, for each level, the amount of time a target spends on-screen and the number of targets that appear simultaneously will vary. The games also provide a leader board for self-assessment corresponding to each attempt by the user.

Optionally, the selected user activity is a self-assessment questionnaire form, and wherein the user input is a health-related information. Optionally, the self-assessment questionnaire form comprises questions relating to an overall health of the user, such as for example motor and non-motor symptoms experienced by the user. The motor symptoms include, for example, mobility, dexterity, pain or discomfort associated with movement, and so forth. The non-motor symptoms include, for example, real-time or historic information such as age, height, weight, body mass index (BMI), diet, sleep levels, rest levels, or stress levels of the user. Beneficially, the self-assessment questionnaire form allows the user to provide health-related information related to the symptoms and progression thereof over a period of time. Moreover, said health-related information could be analysed by the healthcare professional or carer of the user for identifying merits of a current treatment regimen and/or suggesting an alternative treatment regimen. Moreover, the health-related information is provided by selecting a suitable option from an available list of options or providing text information. In this regard, the self-assessment questionnaire form may require the user to choose for example from a predefined scale of responses ranging for example from "None, Slight, Moderate, Severe and Unable" to answer for questions such as "problems with walking", "problems with washing or dressing"," problems with daily activities", "pain/discomfort", "anxiety/depression", and so forth. The application software allows the user to move to a new question after registering an answer for the present question. Moreover, the user interface allows the user to evaluate an overall health (quality of life (QOL)) on a scale of 0 to 100, where 0 is the worst and 100 is the best health of the user.

Optionally, the selected user activity is a charting task, and wherein the user input is at least one of: a medication data, a lifestyle data, a combination therapy data, an appointment log, a medication adherence log. Optionally, the charting task enables recording a status of a prescribed regimen such as the medication data, a dose and frequency thereof, lifestyle data (such as performing an activity such as walking, resting, sleeping, eating), a combination therapy data (such as allopathic treatment, yoga, meditation, naturopathy, homeopathic treatment, and so forth) and frequency thereof, details of upcoming appointment with the healthcare professional, and so on. Moreover, the charting task enables the user to register daily logs for analysis by the healthcare professional or carer of the user. Moreover, the user input is typically selecting a suitable option from an available list of options or providing text information The user input for the charting task comprises feeding into the user device the prescribed regimen along with associated details thereof, such as a dose and frequency of medicine, duration of a physical activity, due appointment, and so forth, and setting suitable notifications for alerting the user to follow the prescribed regimen. In this case, the application software provides an input area to feed prescription information related to, for example, names of medicines, doses of medicines, time of taking medicine, medication next due, scheduling appointments, and so on. Additionally, a log is provided for the user to register an answer (in a Yes or a No) if the said alert was addressed. It will be appreciated that the charting task provides the user with an alert system. Specifically, the alert system is configured to initiate an alarm corresponding to the medication next due and/or scheduled appointment. In this regard, the alert system allows the user to set alarms, provide prescription details (i.e. medicines and doses) and the frequency for each medicine. Moreover, the alert system may further be operable to receive a feedback from the user whether the said alert has been noted and/or addressed.

Moreover, the method comprises operating the server arrangement for analysing the user input and assigning a performance score thereto. Optionally, the user input is analysed for at least one of: a fingertip position, a strength of finger tap, a lateral finger movement, an angular finger movement, an amplitude of finger movement, a regularity of finger movement, a speed of finger movement, a control of finger movement, a spontaneity of finger movement, an accelerometer data, a gesture, a stare, a speech, an amplitude of speech. The fingertip position, strength of the fingertip, an overall movement of the user, voice inputs or speech, and accelerometer data are analysed for assessing level of impact of the medical condition on the user. It will be appreciated that the term *"overall movement"* relates to various aspects of motor movements, such as amplitude, speed, swing, regularity, control, spontaneity (or hesitation or rigidity), angular and lateral movements associated with finger, hands, neck, legs, trunk, or any other body part. Moreover, the speech of the user may be used to assess the confidence levels and/or hesitation in the user to suggest a combination therapy, such as speech therapy, to the user. Optionally, the server arrangement assigns the performance score to each user input, based on the analysis of the user input.

The term *"performance score"* as used herein refers to a quantifier representative of a control while providing the user input. The performance score is typically a number, represented as a fraction of numbers, a decimal number, a percentage, and so forth. The performance score assigned to a given user input is calculated from an analysis of the user input. As mentioned above, the user input is associated with at least one of the health-related information of the user (i.e. motor and non-motor symptoms), the inertial data of the user (i.e. dexterity and speed and accuracy of movement while playing games), the visuomotor feedback, the visual feedback, the vocal input, and/or cognitive value. Moreover, the user input associated with the health-related information corresponding to the self-assessment questionnaire form is assigned a performance score based on a historic data recorded for the user inputs during a predetermined period of time. Furthermore, the user input associated with inertial data, visuomotor feedback, visual feedback, vocal input, and/or cognitive value of the user is assigned a performance score based on several recorded parameters, such as the mean deviation to a guiding point or line and so forth. In an example, the spiral drawing test may be used to capture drawing trajectory for analysis and the performance score is generated based thereon through radial acceleration and the number of velocity peaks (as discussed in *Azulay et. al.,* 2019). In another example, the line drawing test enables more immediate performance scoring by capturing total distance travelled and speed. Typically, a higher performance score is associated with a greater level of control (namely, perfection) of said user input corresponding to the selected user activity. As a result, user input with the higher performance score corresponds to a better control of any of: the motor movement, visuomotor feedback, visual feedback, vocal input, and/or cognitive value as compared to a lower performance score corresponding to a given user input. In an embodiment, the server arrangement assigns performance scores to user inputs based on a participation frequency, namely, the number of times the user selects a given user activity and registers a user input therefor.

Moreover, the method comprises operating the server arrangement for identifying, based on the performance score, one or more symptoms. In this regard, the symptom corresponding to the performance score with a lowest threshold is identified as the symptom the user experiences. The lowest threshold for the performance score corresponds to the weakest control of any of: the motor movement, visuomotor feedback, visual feedback, vocal input, and/or cognitive value corresponding to a given user input. It will be appreciated that the lowest threshold may be associated with a symptom associated with an advanced level of a medical condition. Beneficially, analyzing the performance scores for the lowest threshold thereof enables validating a given symptom experienced by the user.

Optionally, identification of the one or more symptoms based on the performance score, comprises comparing at least two performance scores, and wherein the at least two performance scores correspond to a new user input and a past user input associated with the selected user activity. In this regard, the new user input is received in real-time for a user activity and is assigned a performance score based on the performance score of the past user input corresponding to the same user activity. In an embodiment, more than one user activity may be analysed for identifying the symptom based on the performance scores.

Optionally, the server arrangement is configured to collect, collate and analyse the user input and assign performance score corresponding to the user input corresponding to the selected user activity. Optionally, a plurality of performance scores generated corresponding to the selected user activity are collated and provided unaltered or in the form of a graph to be reported (via a communication network, such as internet, Bluetooth^{®} connection, and so on) to the user, user's healthcare professional or a remote server, for further processing and/or analysis. In this regard, the server arrangement provides a tool for data collection, such as from raw accelerometer or drawing recordings, over a period of time and storing the data in a memory unit of the server arrangement. Optionally, the user input may be used to establish models, for example using machine learning methods, to provide a performance score to user inputs and identify symptoms experienced by the user, by aggregating data from the various user activities. Optionally, the user activities, such as spiral drawing and accelerometer measurements, that require processing may also be attributed. Additionally, or alternatively, the server arrangement triggers an automatic alert when the readings go beyond a predefined threshold. The performance score has numerous potential uses, such as establishing a potential measure to identify a symptom based thereon, manage the progression of symptoms in the user, understand the effect of different biomarkers, medication regimes or environmental factors on the medical condition.

Furthermore, the method comprises operating the server arrangement for assigning a severity score to the symptom for associating the symptom with the medical condition. In this regard, the severity score corresponds to the probability of the symptom to be associated with the medical condition. It will be appreciated that a symptom may be common to one or more medical conditions. Therefore, a high severity score corresponding to the symptom being associated with a given medical condition validates the association of said symptom with the medical condition.

Optionally, the method comprises operating the server arrangement for receiving, from the user device, a self-assessment of symptoms experienced by the user, prior to providing, to the user device, a set of user activities. In this regard, the user may provide a text information or select a suitable option from an available list of options, an audio input, a video input, a graphic input, a gesture, or a combination thereof. Optionally, input corresponding to the self-assessment of symptoms enables the server arrangement to provide, to the user device, only the set of activities that validate a certain type of symptoms. Beneficially, a scrutinized set of user activities reduce the computation power and time associated with analysis of one or more user inputs.

Optionally, the method comprises operating the server arrangement for recommending, to the user device, a subsequent user activity based on the performance score. Optionally, the server arrangement implements a machine learning algorithm to extract information corresponding to preferences for a selected user activity and recommend the subsequent user activity based on the performance score. In this regard, the machine learning algorithm extracts information from at least one of: a clinical validation tool for symptom assessment, a market research, a psychological research, an online source, an in-person interview, a cross-sectional study, a customer survey template. In this regard, the subsequent user activities are recommended to the user device one at a time. Optionally, the subsequent user activity is recommended randomly, and each subsequent user activity may be similar to the selected user activity or a different user activity. More optionally, the subsequent user activity is an advanced level selected from the multiple skill levels of the selected activity or a new user activity different from the selected user activity. Moreover, the method comprises operating the server arrangement for repeating the aforementioned steps of the method, i.e. from receiving information relating to selection of a user activity, receiving a user input associated therewith, analysing the user input and assigning a performance score thereto, and recommending a subsequent user activity based on the performance score, until the performance score is determined as a lowest threshold for the user input associated with the at least one user activity.

Optionally, the method comprises operating the server arrangement for determining, based on at least two performance scores, a progression of the symptom associated with the medical condition. Optionally, the performance score is associated with the progression of symptoms in the user. In an example, a high performance score may be associated with a reduced progression of symptoms, i.e. a good quality of life, while a low performance score may be associated with a deteriorating health of the user. Optionally, determining the progression of the symptom based on the performance score comprises comparing at least two performance scores, and wherein the at least two performance scores correspond to a new user input and a past user input associated with a user activity. In this regard, the user input is received in real-time for a user activity and is assigned a performance score based on the progression of a symptom from a previous recording of or past user input corresponding to the same user activity. In an embodiment, more than one user activity may be analysed to determine the progression of the symptom based on the performance scores.

Optionally, the method further comprises providing a feedback based on the determined progression of the symptom. The feedback includes, but is not limited to, a follow-up with the healthcare professional, user participation in the set of user activities, a change in current physical activity, and so on. The feedback is typically different from the current regimen of the user and is directed at an improved potential performance score based on which a new information related to the progression of the symptom will be determined.

Optionally, the method comprises training the server arrangement using machine learning and artificial intelligence, for analysis of the at least one user input, identification of the one or more symptoms, and recommendation of the subsequent user activity. Herein, training the server arrangement utilizes one or more of user activities and/or subsequent user activities, one or more user inputs, one or more performance scores, one or more symptoms, one or more severity scores, and a database corresponding to a plurality of medical conditions.

Optionally, the methods of the present disclosure may be employed as one of several combination therapy options available, such as medication, deep brain stimulation, and other medical devices, and treatment method using assistive and rehabilitative devices. Furthermore, the methods of the present disclosure allows patients to independently assess their symptoms and monitor progression thereof before or after a clinical diagnosis, while trying their best to maintain high quality of life. Moreover, an aim of the aforementioned system and corresponding method is to improve the daily life of a subject, such as for example a Parkinson's patients, by including features like video call software, dictation software, virtual fitness coaches, calendar and alarms applications, and pill boxes. It will be appreciated that the aforementioned system and method ensure that such tools are developed with a focus on clinicians as well as the user of the system, and improve the ability to receive and process user feedbacks quickly and accurately. Moreover, gamified versions of the clinically validated evaluation tools encourage enhanced participation from the users in between infrequent appointments with clinicians. Furthermore, in between the subsequent appointments, users are encouraged to record their symptoms and daily activities and adhere to the prescribed medication schedules without involving family or carers thereof, thus making the patient independent.

It will be appreciated that the method of the present disclosure is a stand-alone solution for associating the symptom with the medical condition and monitoring progression of the symptoms. Moreover, the user may engage in the set of user activities routinely, such as during an experience of symptoms (such as tremors and slowness, experience of debilitating symptoms), change of medication schedule, and so on, to maintain a high quality of life. Alternatively, the method may be used with conventional therapies (including medicines, meditation, etc.) for associating the symptoms with the medical condition.

The present disclosure also relates to the system as described above. Various embodiments and variants disclosed above apply mutatis mutandis to the system.

Optionally, the user device is communicably coupled to a wearable device, wherein the wearable device is configured to be in contact with the user, when in operation. Optionally, the wearable device is communicably coupled to the user device via any one of: Bluetooth^{®}, near field communication, Wi-Fi. Optionally, the wearable device is configured to be attachable to, detachable from and/or reattachable to a body part of the user via a proximal surface thereof. Optionally, the body part is a skin of: head, sternum, arm, shoulder, wrist, hand, neck, trunk, ankle, leg, thigh, foot, temple, face, neck, elbow, wrist, fingers, spine, knee, and so forth. Alternatively, optionally, the wearable device may be a handheld device or configured to be attachable to, detachable from and/or reattachable to a cloth or a footwear of the user, via a proximal surface thereof. Optionally, the physical contact with the user is by a mechanical engagement means, selected from at least one of: an adhesive, a strap, a locket, a bracelet, a band, a belt, a vacuum cup, a magnet, and a hook and loop fastener. It will be appreciated that the wearable device is wearable over any body part of the user or placed at a suitable location, such as on a shirt, footwear, headband, and so on, rather than a slip-on arrangement. For example, the wearable device may be placed upon the skin directly (for example using adhesives, vacuum cups, and so on) or incorporated into one of a bracelet, a pendant, an anklet, an arm band, a wrist band, clothes, footwear, or other item such that the proximal surface thereof is arranged facing the skin of the user. The wearable device is designed to sense at least one symptom experienced by the user, and/or provide symptomatic relief from the symptom. Optionally, the wearable device is used in addition to taking a prescribed medication, to help monitor and/or alleviate symptoms experienced by the user. Alternatively, optionally, the wearable device is a stand-alone solution to help monitor and/or alleviate symptoms experienced by the user.

Optionally, the wearable device comprises a stimulating element, and wherein the stimulating element is configured to control the symptom. Optionally, the stimulating element transmits at least one of: an electrical stimulus, a mechanical stimulus, and an electro-mechanical stimulus to manage the symptom, such as an abnormal involuntary movement, of the user. Optionally, the stimulus is or comprises a periodic output, continuous output or pulsed output. Optionally, the wearable device comprises a dissipating portion configured to increase an effective area for the stimulating element to dissipate the stimulus to the user. Optionally, the wearable device is in contact with the user via the dissipating portion. Optionally, the wearable device comprises an electric charging portion to supply an onboard battery which powers the wearable device. Optionally, the electric charging portion is configured to receive electric power (for example electric power) from an external electric power source by a wireless or wired connection, for example a wireless resonant-inductive connection. Optionally, the wearable device comprises a modulation means configured to modulate the operating parameter associated with the stimulating element. The operating parameters associated with the stimulating element may include such as an intensity, a frequency, an amplitude and a phase of the stimulus. Optionally, the wearable device comprises an alerting module, an LED module, and a sound module. Optionally, the wearable device comprises a controller configured to control an output of the wearable device.

Optionally, the wearable device is configured to receive, via the user device, a user instruction for customizing an operating parameter associated with the stimulating element. Optionally, the user instruction comprises one or more commands to instruct at least one component of the wearable device, such as the stimulating element, the battery, the modulation means, the alerting module, the LED module, the sound module, the sensor, and so on, to control the operation of the wearable device.

Optionally, the wearable device comprises a sensor operable to detect a symptom experienced by the user. The sensor typically detects a symptom such as an abnormal involuntary movement, for example a tremor, a shaking, and the like. Optionally, the sensor is selected from at least one of: a gyroscopic sensor, an accelerometer sensor, a magnetometer sensor, an electromyography sensor, a flexural sensor, a stretch sensor, or a combination thereof.

Optionally, the server arrangement, associated with the user device, is configured to receive a sensor data from the wearable device; analyze the change in operation of the wearable device; and provide an instruction to the wearable device to control operation thereof based on the analyzed data. The server arrangement is operable to store and use historic data in a memory unit thereof. The historic data is typically a log of instructions provided in the past to modulate the operations of the wearable device in a similar event. The historic data is typically associated with a change in operation of the wearable device, such as a change in stimulation.

Optionally, the user interface of the user device allows the user to modulate the operating parameters of the wearable device. Optionally, the user interface enables the user to apply touch or light pressure thereon to access the wearable device when in operation. The touch or light pressure application by the user on the user interface is configured to provide instructions to any of: the controller or modulation means to instruct the one or more components of the wearable device to provide an output based on a user response for controlling the operation of the wearable device. In an example, the user response is directed at selecting a range of intensities of stimulation to manage symptoms experienced by the user. In another example, the user response is directed at medication alerts, advanced notification for scheduling appointments with the doctor, reminders for regularly using the wearable device, and so on.

In an alternate embodiment, the wearable device is operable to be used without any Bluetooth^{®} connection. In some embodiments, the server arrangement is used to trigger the wearable device from a remote location, for example by the user, or the user's doctor, carer, or a family member authorised to access the wearable device in an emergency situation.

The present disclosure also relates to the computer program product as described above. Various embodiments and variants disclosed above apply mutatis mutandis to the computer program product.

The computer program product comprises a non-transitory computer-readable storage medium having computer-readable instructions stored thereon, the computer-readable instructions being executable by a computerized device comprising processing hardware to execute the aforementioned method.

### DETAILED DESCRIPTION OF THE DRAWINGS

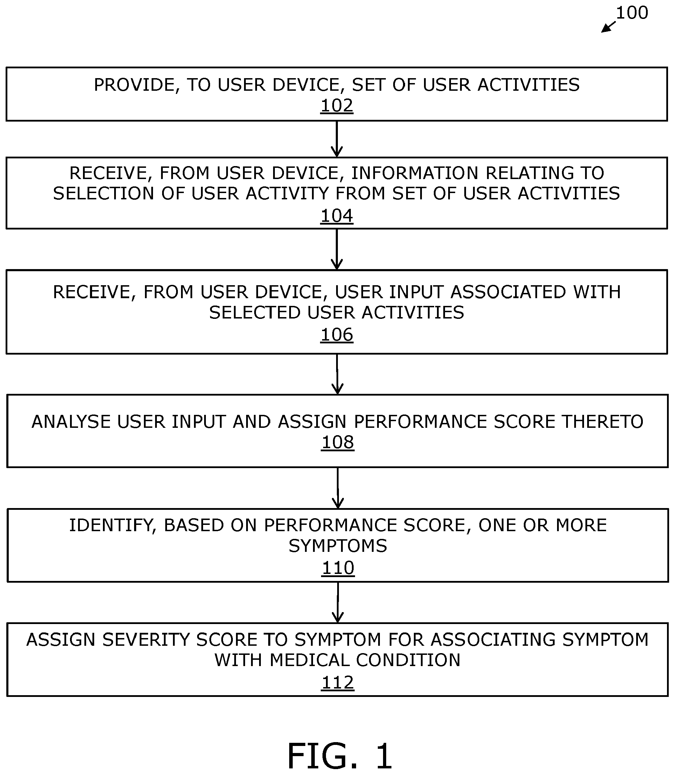

Referring to FIG. 1, shown is a flowchart **100** of steps of a method for associating a symptom with a medical condition. At step **102,** a set of user activities is provided to a user device. At step **104,** information relating to selection of a user activity from the set of user activities is received from the user device. At step **106,** a user input associated with the selected user activity is received from the user device. At step **108,** the user input is analysed and a performance score is assigned thereto. At step **110,** one or more symptoms are identified based on the performance score. At step **112,** a severity score is assigned to the symptom for associating the symptom with a medical condition.

The steps **102, 104, 106, 108, 110** and **112**are only illustrative and other alternatives can also be provided where one or more steps are added, one or more steps are removed, or one or more steps are provided in a different sequence without departing from the scope of the claims herein.

Referring to FIG. 2, illustrated is an exemplary illustration of a system **200** for associating a symptom with a medical condition. As shown, the application software is successfully installed as a user interface **202** on the display of the user device **204.** The user interface **202** allows providing (by means of displaying) a set of user activities and/or controlling operations of a wearable device **206** when in use by a user **208.** The user interface **202** receives selection of a user activity from the set of user activities from the user **208.** The user device **204** and the wearable device **206** are communicably coupled via a communication network **210.** The user interface **202** enables sending the data recorded by the user **208** for further analysis by a physician, a carer, or a server and for tracking the progress of the user **208.**

Referring to FIGs. 3, 4A, 4B, 4C, 5A, 5B, 5C, 6A and 6B, illustrated are exemplary user interfaces **202** of the user device **204.**

As shown in FIG. 3, a logo or icon **302** of the user interface **202** is visible on the display of the user device **204.** The user interface **202** comprises a main menu **304** with a plurality of options, such as **304A, 304B** (such as a variety of games (such as motor gaming tasks, voice-controlled gaming tasks), self-assessment questionnaire form, charting task, and so on). The user interface **202** allows toggling between the various options, such as **304A, 304B,** on the main menu **304** by going back from one option, such as **304A,** to the main menu **304** and selecting another option, such as **304B.**

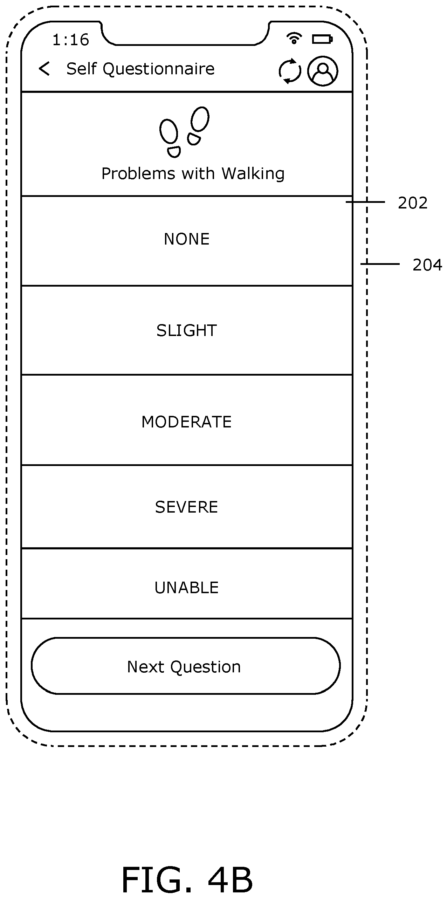

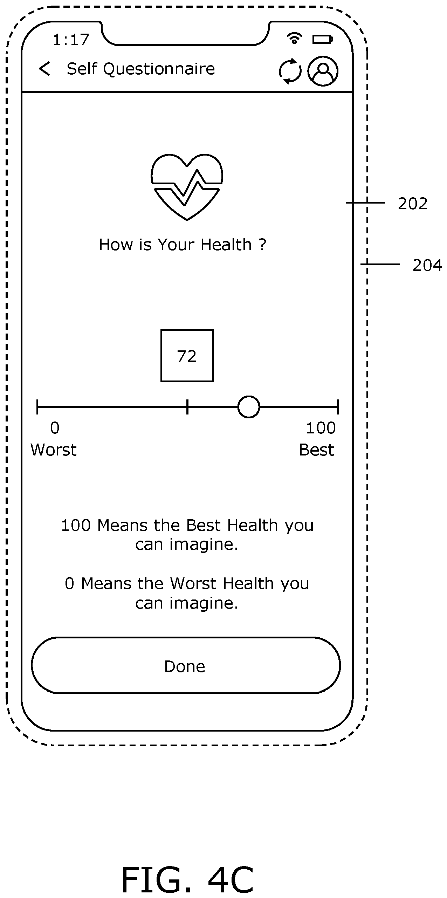

As shown in FIG. 4A, the user interface **202** allows the user to monitor or submit for further analysis, to a doctor, a carer, or a server for example, the progress of disease by filling out a form comprising a set of questions, such as via a self-assessment questionnaire form. As shown in FIG. 4B, the self-assessment questionnaire form provides a predefined scale of responses ranging for example from "None, Slight, Moderate, Severe and Unable" to answer for questions such as "problems with walking", "problems with washing or dressing"," problems with daily activities", "pain/discomfort", "anxiety/depression", and so forth for a user to select from corresponding to a real-time condition of the user. Moreover, the user interface **202** allows the user to move to a new question after registering an answer for the present question. As shown in FIG. 4C, the user interface **202** allows the user to evaluate an overall health (quality of life (QOL)) on a scale of 0 to 100, where 0 is the worst and 100 is the best health the user can imagine.

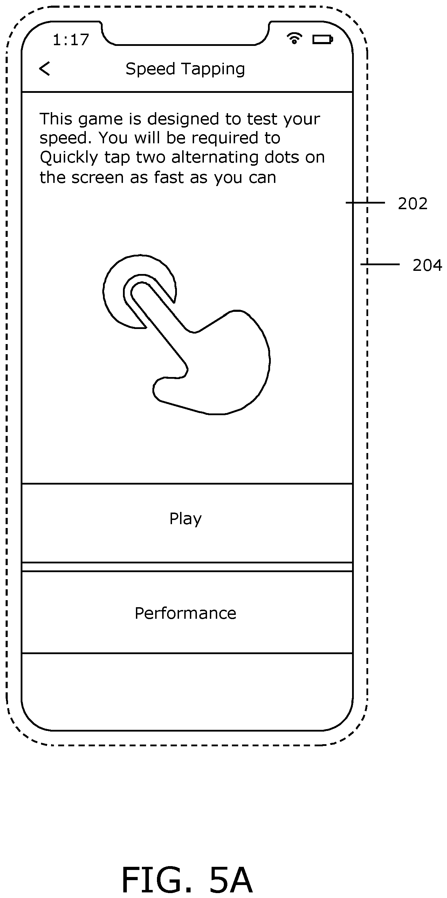

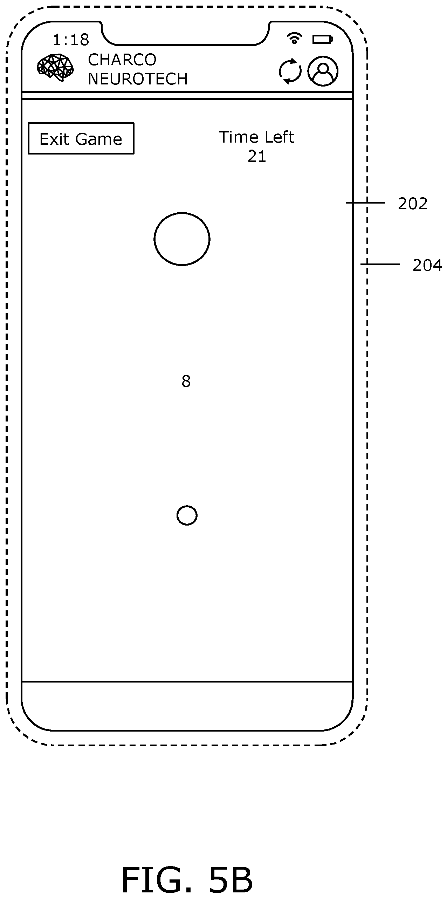

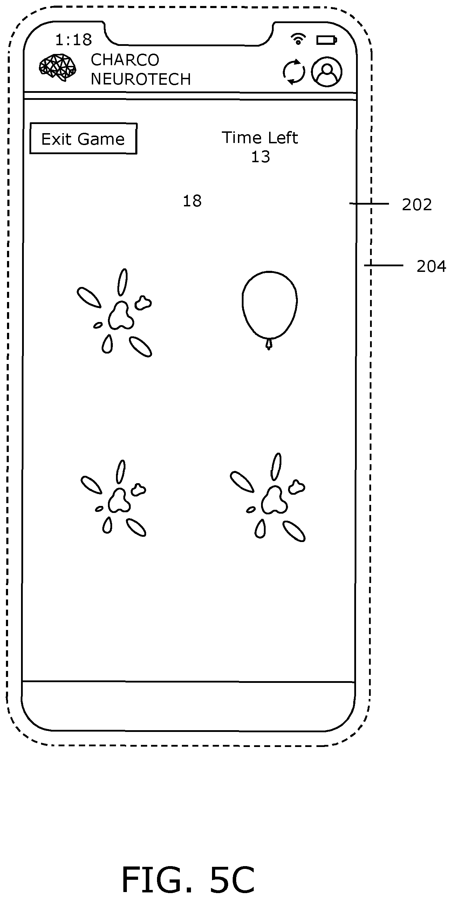

As illustrated in FIG. 5A, the user interface **202** allows the user to select from a variety of games designed to test dexterity and speed of the user. As shown in FIG. 5B, one of the variety of games include drawing a spiral and/or a straight line between the two points shown on the screen using a finger or a stylus pen. As shown in FIG. 5C, one of the variety of games include identifying and tracking a change in the display of the user device **204,** for example, hitting a target, such as popping balloons, within a time limit. The user can take multiple attempts that help in improving dexterity and focus, and enable fighting isolation.

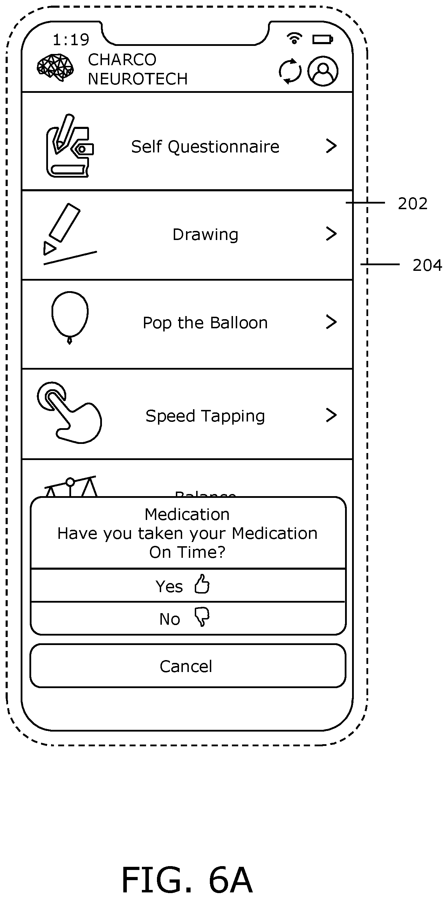

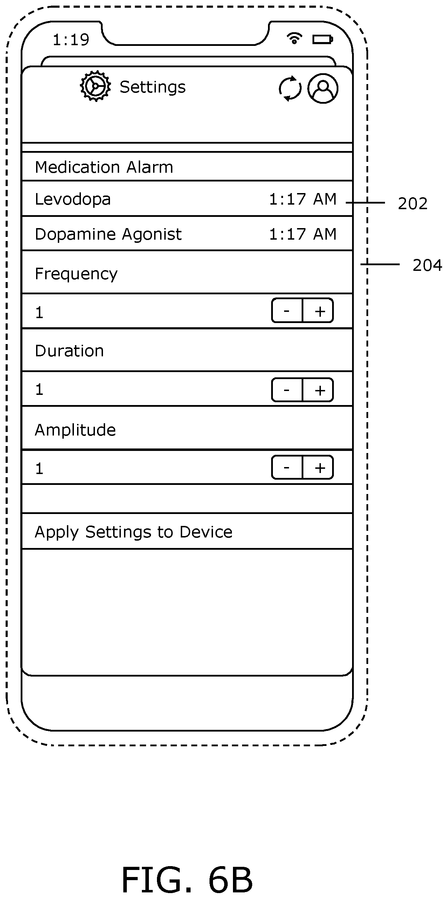

As shown in FIG. 6A, the user interface **202** provides charting tasks, such as the medication next due. Additionally, a log is provided for the user to register an answer (in a Yes or a No) if the said alert was addressed and the medication was taken due for that time. As shown in FIG. 6B, a Settings icon allows the user to set alarms, provide prescription details (i.e. medicines and doses) and the frequency for each medicine.

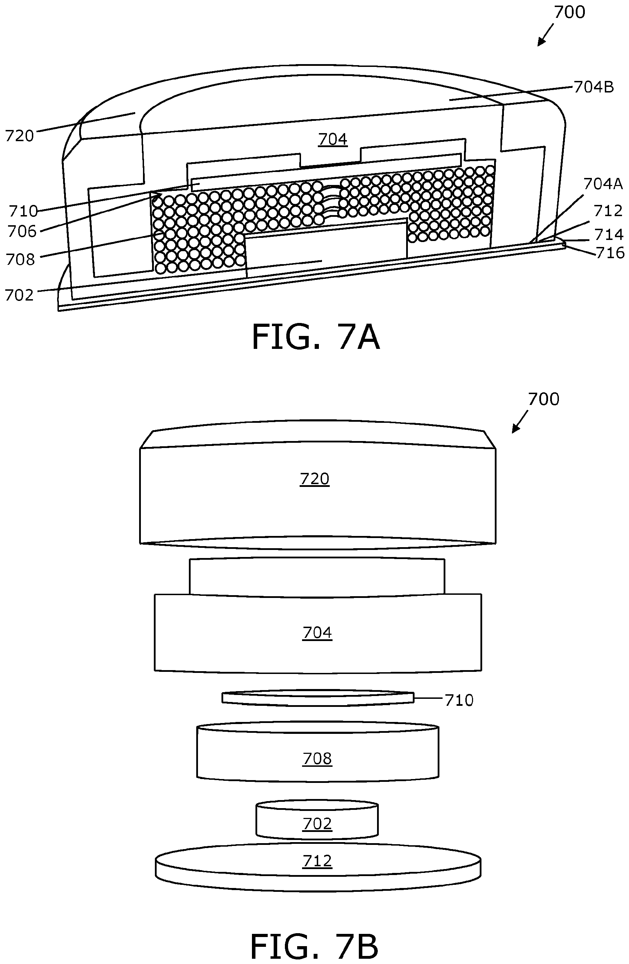

Referring to FIG. 7A and 7B, illustrated are a cross-sectional view and an exploded view of an exemplary wearable device **700** respectively, in accordance with an embodiment of the invention. As shown in Figure 7A, the wearable device comprises a sensor (not shown), a stimulating element **702** and a dissipating portion **704** having a proximal end **704A** and a distal end **704B.** The dissipating portion **704** comprises a recess **706** configured to receive the stimulating element **702,** a charging portion **708,** and a controller **710.** The dissipating portion **704** forms at least a part of the surface **712** for providing stimulus to the user as well as surface for contacting the user. The ring-shaped area is configured to receive an adhesive **714** which in turn enables the wearable device **700** to be adhered to the user. In the embodiment shown, the adhesive **714** is substantially level with the proximal end **704A** of the dissipating portion **704** and the stimulating element **702** forming the surface **712.** The wearable device further comprises a housing **716.** As shown in FIG. 7B, the exploded view of the wearable device **700** comprises stimulating element **702,** a dissipating portion **704,** a charging portion **708,** a controller **710,** a surface **712** for providing stimulus, and a housing **716.**

Modifications to embodiments of the present disclosure described in the foregoing are possible without departing from the scope of the present disclosure as defined by the accompanying claims.

## Claims

1. A system (200) for associating a symptom with a medical condition, the system comprising a server arrangement, associated with a user device (204), the server arrangement is operable to:
(a) provide, to the user device (204), a set of user activities;
(b) receive, from the user device (204), information relating to a selection of a user activity from the set of user activities;
(c) receive, from the user device (204), a user input associated with the selected user activity;
(d) analyse the user input and assign a performance score thereto;
(e) identify, based on the performance score, one or more symptoms; and
(f) assign a severity score to the symptom for associating the symptom with a medical condition,
**characterized in that**, the identification of the one or more symptoms based on the performance score, comprises comparing at least two performance scores, wherein the at least two performance scores correspond to a new user input and a past user input associated with the selected user activity.

2. The system (200) of claim 1, wherein the server arrangement is further operable to (g) recommend, to the user device (204), a subsequent user activity based on the performance score; and (h) repeat steps (b) to (d) and (g) until the performance score is determined as a lowest threshold for the user input associated with the at least one user activity.

3. The system (200) of any one of the preceding claims, wherein the server arrangement is trained using machine learning and artificial intelligence, for analysis of the at least one user input, identification of the one or more symptoms, and recommendation of the subsequent user activity.

4. The system (200) of any one of the preceding claims, wherein the selected user activity is a motor gaming task, that requires the user input as a motor movement, and wherein the motor gaming task is selected from at least one of: a speed tapping game, a balancing game, a maze game, a puzzle game, an interactive cognitive game, a cognitive task;
or wherein the selected user activity is a voice-controlled gaming task that requires the user input as at least one of: a vocal input, a motor movement;
or wherein the selected user activity is a self-assessment questionnaire form and the user input is a health-related information;
or wherein the selected user activity is a charting task, and wherein the user input is at least one of: a medication data, a lifestyle data, a combination therapy data, an appointment log, a medication adherence log.

5. The system (200) of any one of the preceding claims, wherein the medical condition is associated with at least one of: a neurological disorder, a muscular disorder, a neuro-muscular disorder;
and/or wherein the medical condition is Parkinson's disease;
and/or wherein the symptom associated with the medical condition is at least one of: a bradykinesia, a dyskinesia, a tremor, an akinesia, a hypokinesia, a micrographia, a hypophonia, a tachyphemia.

6. The system (200) of any one of the preceding claims, wherein the user input is analysed for at least one of: a fingertip position, a strength of finger tap, a lateral finger movement, an angular finger movement, an amplitude of finger movement, a regularity of finger movement, a speed of finger movement, a control of finger movement, a spontaneity of finger movement, an accelerometer data, a movement of the phone, a gesture, a stare, a speech, an amplitude of speech.

7. The system (200) of any one of the preceding claims, wherein the server arrangement is further configured to determine, based on at least two performance scores, a progression of the symptom associated with the medical condition.

8. The system (200) of any one of the preceding claims, wherein the user device (204) is communicably coupled to a wearable device (700), wherein the wearable device (700) is configured to be in contact with the user (208), when in operation.

9. The system (200) of claim 8, wherein the wearable device (700) comprises a stimulating element, and wherein the stimulating element is configured to control the symptom.

10. The system (200) of claim 8 or 9, wherein the wearable device (700) is configured to receive, via the user device (204), a user instruction for customizing an operating parameter associated with the stimulating element.

11. A computer program product comprising a non-transitory computer-readable storage medium having computer-readable instructions stored thereon, the computer-readable instructions being executable by a computerized device comprising processing hardware to execute a method (100) for associating a symptom with a medical condition using a system of any of the preceding claims, the method comprising operating a server arrangement, associated with a user device (204), for:
(a) providing, to the user device (204), a set of user activities;
(b) receiving, from the user device (204), information relating to selection of a user activity from the set of user activities;
(c) receiving, from the user device (204), a user input associated with the selected user activity;
(d) analysing the user input and assigning a performance score thereto;
(e) identifying, based on the performance score, one or more symptoms; and
(f) assigning a severity score to the symptom for associating the symptom with a medical condition,
**characterized in that**, the identification of the one or more symptoms based on the performance score, comprises comparing the at least two performance scores, and wherein the at least two performance scores correspond to a new user input and a past user input associated with the selected user activity.

12. The computer program product of claim 11, wherein the method further comprises operating the server arrangement for
(g) recommending, to the user device (204), a subsequent user activity based on the performance score; and
(h) repeating steps (b) to (d) and (g) until the performance score is determined as a lowest threshold for the user input associated with the at least one user activity.

13. The computer program product of claim 11 or 12, wherein the method comprises training the server arrangement using machine learning and artificial intelligence, for analysis of the at least one user input, identification of the one or more symptoms, and recommendation of the subsequent user activity.

14. The computer program product of any one of claims 11 to 13, wherein the method further comprises operating the server arrangement to determine, based on at least two performance scores, a progression of the symptom associated with the medical condition.

## Patentansprüche

1. System (200) zum Assoziieren eines Symptoms mit einer Krankheit, wobei das System eine Serveranordnung umfasst, die mit einem Benutzergerät (204) assoziiert ist, wobei die Serveranordnung betreibbar ist, um:
(a) einen Satz von Benutzeraktivitäten an das Benutzergerät (204) bereitzustellen;
(b) Informationen von dem Benutzergerät (204) zu empfangen, die sich auf eine Auswahl einer Benutzeraktivität aus dem Satz von Benutzeraktivitäten beziehen;
(c) eine Benutzereingabe von dem Benutzergerät (204) zu empfangen, die mit der ausgewählten Benutzeraktivität assoziiert ist;
(d) die Benutzereingabe zu analysieren und ihr einen Leistungswert zuzuweisen;
(e) eines oder mehrerer Symptome basierend auf dem Leistungswert zu identifizieren; und
(f) eine Schweregradwerts zu dem Symptom zuzuweisen, um das Symptom mit einer Krankheit zu assoziieren,
**dadurch gekennzeichnet, dass** die Identifikation des einen oder der mehreren Symptome basierend auf dem Leistungswert das Vergleichen von mindestens zwei Leistungswerten umfasst, wobei die mindestens zwei Leistungswerte einer neuen Benutzereingabe und einer vergangenen, mit der ausgewählten Benutzeraktivität assoziierten Benutzereingabe entsprechen.

2. System (200) nach Anspruch 1, wobei die Serveranordnung ferner betreibbar ist, um (g) dem Benutzergerät (204) eine nachfolgende Benutzeraktivität basierend auf dem Leistungswert zu empfehlen; und (h) die Schritte (b) bis (d) und (g) zu wiederholen, bis der Leistungswert als ein niedrigster Schwellenwert für die mit der mindestens einen Benutzeraktivität assoziierte Benutzereingabe bestimmt wird.

3. System (200) nach einem der vorhergehenden Ansprüche, wobei die Serveranordnung unter Verwendung von Maschinenlernen und künstlicher Intelligenz zur Analyse der mindestens einen Benutzereingabe, zur Identifikation des einen oder der mehreren Symptome und zur Empfehlung der nachfolgenden Benutzeraktivität trainiert wird.

4. System (200) nach einem der vorhergehenden Ansprüche, wobei die ausgewählte Benutzeraktivität eine Motorspielaufgabe ist, die die Benutzereingabe als eine Motorbewegung erfordert, und wobei die Motorspielaufgabe ausgewählt ist aus mindestens einem von: einem Klopfgeschwindigkeitsspiel, einem Balancespiel, einem Labyrinthspiel, einem Puzzlespiel, einem interaktiven kognitiven Spiel, einer kognitiven Aufgabe;
oder wobei die ausgewählte Benutzeraktivität eine sprachgesteuerte Spielaufgabe ist, die die Benutzereingabe als mindestens eines der Folgenden erfordert: einer Spracheingabe, einer Motorbewegung;
oder wobei die ausgewählte Benutzeraktivität ein Selbstbewertungsformular ist und die Benutzereingabe eine gesundheitsbezogene Information ist;
oder wobei die ausgewählte Benutzeraktivität eine Diagrammerstellungsaufgabe ist, und wobei die Benutzereingabe mindestens eines ist von: Medikationsdaten, Lebensstildaten, Kombinationstherapiedaten, einem Terminprotokoll, einem Medikationseinhaltungsprotokoll.

5. System (200) nach einem der vorangehenden Ansprüche, wobei die Kranheit assoziiert ist mit zumindest einem von: einer neurologischen Störung, einer Muskelstörung, einer neuromuskulären Störung;
und/oder wobei die Krankheit die Parkinson-Krankheit ist;
und/oder wobei das Symptom, das mit der Kranheit assoziiert ist, mindestens eines ist von: einer Bradykinesie, einer Dyskinesie, einem Tremor, einer Akinesie, einer Hypokinesie, einer Mikrographie, einer Hypophonie, einer Tachyphämie.

6. System (200) nach einem der vorhergehenden Ansprüche, wobei die Benutzereingabe für mindestens eines der Folgenden analysiert wird: einer Fingerspitzenposition, einer Fingerklopfstärke, einer seitlichen Fingerbewegung, einer winkelförmigen Fingerbewegung, einer Amplitude einer Fingerbewegung, einer Regelmäßigkeit einer Fingerbewegung, einer Geschwindigkeit einer Fingerbewegung, einer Steuerung einer Fingerbewegung, einer Spontaneität einer Fingerbewegung, Beschleunigungsmessdaten, einer Bewegung des Telefons, einer Geste, einem Blick, einer Sprache, einer Amplitude der Sprache.

7. System (200) nach einem der vorhergehenden Ansprüche, wobei die Serveranordnung ferner konfiguriert ist, um basierend auf mindestens zwei Leistungswerten ein Fortschreiten des Symptoms zu bestimmen, das mit der Krankheit assoziiert ist.

8. System (200) nach einem der vorhergehenden Ansprüche, wobei das Benutzergerät (204) mit einer tragbaren Vorrichtung (700) kommunikativ gekoppelt ist, wobei die tragbare Vorrichtung (700) konfiguriert ist, um im Betrieb mit dem Benutzer (208) in Kontakt zu sein.

9. System (200) nach Anspruch 8, wobei die tragbare Vorrichtung (700) ein stimulierendes Element umfasst, und wobei das stimulierende Element konfiguriert ist, um das Symptom zu steuern.

10. System (200) nach Anspruch 8 oder 9, wobei die tragbare Vorrichtung (700) konfiguriert ist, um über das Benutzergerät (204) eine Benutzeranweisung zum individuellen Anpassen eines Betriebsparameters zu empfangen, der mit dem Stimulationselement assoziiert ist.

11. Computerprogrammprodukt, das ein nichtflüchtiges computerlesbares Speichermedium mit darauf gespeicherten computerlesbaren Anweisungen umfasst, wobei die computerlesbaren Anweisungen durch ein computergestütztes Gerät, das Verarbeitungshardware umfasst, ausführbar sind, um ein
Verfahren (100) auszuführen zum Assoziieren eines Symptoms mit einer Krankheit unter Nutzung eines Systems nach einem der vorhergehenden Ansprüche, wobei das Verfahren das Betreiben einer Serveranordnung umfasst, die mit einem Benutzergerät (204) assoziiert ist, für das:
(a) Bereitstellen eines Satzes von Benutzeraktivitäten an das Benutzergerät (204);
(b) Empfangen von Informationen, die sich auf die Auswahl einer Benutzeraktivität aus dem Satz von Benutzeraktivitäten beziehen, von dem Benutzergerät (204);
(c) Empfangen einer Benutzereingabe, die mit der ausgewählten Benutzeraktivität assoziiert ist, von dem Benutzergerät (204);
(d) Analysieren der Benutzereingabe und Zuweisen eines Leistungswerts zu dieser;
(e) Identifizieren eines oder mehrerer Symptome basierend auf dem Leistungswert; und
(f) Zuweisen eines Schweregradwerts zu dem Symptom, um das Symptom mit einer Krankheit zu assoziieren,
**dadurch gekennzeichnet, dass** die Identifikation des einen oder der mehreren Symptome basierend auf dem Leistungswert das Vergleichen von mindestens zwei Leistungswerten umfasst, wobei die mindestens zwei Leistungswerte einer neuen Benutzereingabe und einer vergangenen, mit der ausgewählten Benutzeraktivität assoziierten Benutzereingabe entsprechen.

12. Computerprogrammprodukt nach Anspruch 11, wobei das Verfahren ferner das Betreiben der Serveranordnung umfasst für das
(g) Empfehlen einer nachfolgenden Benutzeraktivität basierend auf dem Leistungswert an das Benutzergerät (204); und
(h) Wiederholen der Schritte (b) bis (d) und (g), bis der Leistungswert als ein niedrigster Schwellenwert für die Benutzereingabe bestimmt wird, die mit der mindestens einen Benutzeraktivität assoziiert ist.

13. Computerprogrammprodukt nach Anspruch 11 oder 12, wobei das Verfahren das Trainieren der Serveranordnung unter Verwendung von maschinellem Lernen und künstlicher Intelligenz zur Analyse der mindestens einen Benutzereingabe, zur Identifikation des einen oder der mehreren Symptome und zur Empfehlung der nachfolgenden Benutzeraktivität umfasst.

14. Computerprogrammprodukt
nach einem der Ansprüche 11 bis 13, wobei das Verfahren ferner das Betreiben der Serveranordnung umfasst, um basierend auf mindestens zwei Leistungswerten eine Progression des Symptoms zu bestimmen, das mit der Krankheit assoziiert ist.

## Revendications

1. Système (200) pour associer un symptôme à une affection médicale, le système comprenant un agencement de serveur, associé à un dispositif utilisateur (204), l'agencement de serveur étant capable de fonctionner pour :
(a) fournir, au dispositif utilisateur (204), un ensemble d'activités d'utilisateur ;
(b) recevoir, à partir du dispositif utilisateur (204), des informations relatives à une sélection d'une activité d'utilisateur parmi l'ensemble d'activités d'utilisateur ;
(c) recevoir, à partir du dispositif utilisateur (204), une entrée d'utilisateur associée à l'activité d'utilisateur sélectionnée ;
(d) analyser l'entrée d'utilisateur et lui attribuer un score de performances ;
(e) identifier, sur la base du score de performances, un ou plusieurs symptômes ; et
(f) attribuer un score de gravité au symptôme afin d'associer le symptôme à une affection médicale,
**caractérisé en ce que**, l'identification des un ou plusieurs symptômes sur la base du score de performances, comprend la comparaison d'au moins deux scores de performances, dans lequel les au moins deux scores de performances correspondent à une nouvelle entrée d'utilisateur et une entrée d'utilisateur passée associée à l'activité d'utilisateur sélectionnée.

2. Système (200) selon la revendication 1, dans lequel l'agencement de serveur est en outre capable de fonctionner pour (g) recommander, au dispositif utilisateur (204), une activité d'utilisateur ultérieure sur la base du score de performances ; et (h) répéter les étapes (b) à (d) et (g) jusqu'à ce que le score de performances soit déterminé comme un seuil le plus bas pour l'entrée d'utilisateur associée à l'au moins une activité d'utilisateur.

3. Système (200) selon l'une quelconque des revendications précédentes, dans lequel l'agencement de serveur est entraîné à l'aide de l'apprentissage automatique et de l'intelligence artificielle, pour l'analyse de l'au moins une entrée d'utilisateur, l'identification des un ou plusieurs symptômes et la recommandation de l'activité d'utilisateur ultérieure.

4. Système (200) selon l'une quelconque des revendications précédentes, dans lequel l'activité d'utilisateur sélectionnée est une tâche de jeu motrice, qui nécessite l'entrée d'utilisateur sous la forme d'un mouvement moteur, et dans lequel la tâche de jeu motrice est sélectionnée parmi au moins l'un parmi : un jeu de tapotement rapide, un jeu d'équilibre, un jeu de labyrinthe, un jeu de réflexion, un jeu cognitif interactif, une tâche cognitive ;
ou dans lequel l'activité d'utilisateur sélectionnée est une tâche de jeu à commande vocale qui nécessite l'entrée d'utilisateur sous la forme d'au moins l'un parmi : une entrée vocale, un mouvement moteur ;
ou dans lequel l'activité d'utilisateur sélectionnée est un formulaire de questionnaire d'auto-évaluation et l'entrée d'utilisateur est une information liée à la santé ;
ou dans lequel l'activité d'utilisateur sélectionnée est une tâche de consignation, et dans lequel l'entrée d'utilisateur est au moins l'un parmi : des données de médication, des données de mode de vie, des données de thérapie combinée, un journal de rendez-vous, un journal d'observation de médication.

5. Système (200) selon l'une quelconque des revendications précédentes, dans lequel l'affection médicale est associée à au moins l'un parmi : un trouble neurologique, un trouble musculaire, un trouble neuromusculaire ;
et/ou dans lequel l'affection médicale est la maladie de Parkinson ;
et/ou dans lequel le symptôme associé à l'affection médicale est au moins l'un parmi : une bradykinésie, une dyskinésie, un tremblement, une akinésie, une hypokinésie, une micrographie, une hypophonie, une tachyphémie.

6. Système (200) selon l'une quelconque des revendications précédentes, dans lequel l'entrée d'utilisateur est analysée pour au moins l'un parmi : une position de bout de doigt, une force de tapotement de doigt, un mouvement latéral de doigt, un mouvement angulaire de doigt, une amplitude de mouvement de doigt, une régularité de mouvement de doigt, une vitesse de mouvement de doigt, un contrôle de mouvement de doigt, une spontanéité de mouvement de doigt, une donnée d'accéléromètre, un mouvement du téléphone, un geste, un regard, une parole, une amplitude de parole.

7. Système (200) selon l'une quelconque des revendications précédentes, dans lequel l'agencement de serveur est en outre configuré pour déterminer, sur la base d'au moins deux scores de performances, une progression du symptôme associé à l'affection médicale.

8. Système (200) selon l'une quelconque des revendications précédentes, dans lequel le dispositif utilisateur (204) est couplé en communication à un dispositif portable (700), dans lequel le dispositif portable (700) est configuré pour être en contact avec l'utilisateur (208), lorsqu'il est en fonctionnement.

9. Système (200) selon la revendication 8, dans lequel le dispositif portable (700) comprend un élément de stimulation, et dans lequel l'élément de stimulation est configuré pour contrôler le symptôme.

10. Système (200) selon la revendication 8 ou 9, dans lequel le dispositif portable (700) est configuré pour recevoir, par le biais du dispositif utilisateur (204), une instruction d'utilisateur pour personnaliser un paramètre de fonctionnement associé à l'élément de stimulation.

11. Produit-programme d'ordinateur comprenant un support de stockage non transitoire lisible par ordinateur sur lequel sont stockées des instructions lisibles par ordinateur, les instructions lisibles par ordinateur étant exécutables par un dispositif informatisé comprenant un matériel de traitement pour exécuter un procédé (100) pour associer un symptôme à une affection médicale à l'aide d'un système de l'une quelconque des revendications précédentes, le procédé comprenant le la mise en fonctionnement d'un agencement de serveur, associé à un dispositif utilisateur (204), pour :
(a) fournir, au dispositif utilisateur (204), un ensemble d'activités d'utilisateur ;
(b) recevoir, à partir du dispositif utilisateur (204), des informations relatives à la sélection d'une activité d'utilisateur parmi l'ensemble d'activités d'utilisateur ;
(c) recevoir, à partir du dispositif utilisateur (204), une entrée d'utilisateur associée à l'activité d'utilisateur sélectionnée ;
(d) analyser l'entrée d'utilisateur et lui attribuer un score de performances ;
(e) identifier, sur la base du score de performances, un ou plusieurs symptômes ; et
(f) attribuer un score de gravité au symptôme afin d'associer le symptôme à une affection médicale,
**caractérisé en ce que**, l'identification des un ou plusieurs symptômes sur la base du score de performances, comprend la comparaison des au moins deux scores de performances, et dans lequel les au moins deux scores de performances correspondent à une nouvelle entrée d'utilisateur et une entrée d'utilisateur passée associée à l'activité d'utilisateur sélectionnée.

12. Produit-programme d'ordinateur selon la revendication 11, dans lequel le procédé comprend en outre la mise en fonctionnement de l'agencement de serveur pour
(g) recommander, au dispositif utilisateur (204), une activité d'utilisateur ultérieure sur la base du score de performances ; et
(h) répéter les étapes (b) à (d) et (g) jusqu'à ce que le score de performances soit déterminé comme étant un seuil le plus bas pour l'entrée d'utilisateur associée à l'activité d'utilisateur.

13. Produit-programme d'ordinateur selon la revendication 11 ou 12, dans lequel le procédé comprend l'entraînement de l'agencement de serveur à l'aide de l'apprentissage automatique et de l'intelligence artificielle, pour l'analyse de l'au moins une entrée d'utilisateur, l'identification des un ou plusieurs symptômes et la recommandation de l'activité d'utilisateur ultérieure.

14. Produit-programme d'ordinateur
selon l'une quelconque des revendications 11 à 13, dans lequel le procédé comprend en outre la mise en fonctionnement de l'agencement de serveur pour déterminer, sur la base d'au moins deux scores de performances, une progression du symptôme associé à l'affection médicale.
